(19) Europäisches Patentamt
European Patent Office
Office européen des brevets

(11) Veröffentlichungsnummer: **0 185 161**
**B1**

(12) # EUROPÄISCHE PATENTSCHRIFT

(45) Veröffentlichungstag der Patentschrift: **23.11.88**

(51) Int. Cl.⁴: **C 08 F 8/34**, C 12 P 19/44

(21) Anmeldenummer: **85112986.6**

(22) Anmeldetag: **14.10.85**

(54) **Makroporöse Perlpolymerisate zur Reinigung von Acarbose.**

(30) Priorität: **25.10.84 DE 3439008**

(43) Veröffentlichungstag der Anmeldung: **25.06.86 Patentblatt 86/26**

(45) Bekanntmachung des Hinweises auf die Patenterteilung: **23.11.88 Patentblatt 88/47**

(84) Benannte Vertragsstaaten: **AT BE CH DE FR GB IT LI NL SE**

(56) Entgegenhaltungen:
**DE-A-2 719 912**
**DE-B-1 006 613**
**DE-B-1 150 529**
**GB-A-775 539**
**GB-A-989 212**

**Ullmanns Encyklopädie der technischen Chemie, 4. Auflage, Band 19, pp. 2-4**

(73) Patentinhaber: **BAYER AG, Konzernverwaltung RP Patentabteilung, D-5090 Leverkusen 1 Bayerwerk (DE)**

(72) Erfinder: **Lange, Peter Michael, Dr., Walter- Flex-Strasse 9, D-5090 Leverkusen 1 (DE)**
Erfinder: **Rauenbusch, Erich, Dr., Tersteegenweg 9, D-5600 Wuppertal 1 (DE)**

**EP 0 185 161 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft makroporöse vernetzte Perlpolymerisate bzw. polymere Kationenaustauscher auf der Basis von Aromaten mit einer und mit mehreren Vinylgruppe(n) und hydrophilen Monomeren, ein Verfahren zu ihrer Herstellung sowie ihre Verwendung zur Reinigung von Acarbose.

Acarbose ist chemisch eine 0-{4,6-Dideoxy-4-[[1S-(1,4,6/5)-4,5,6-trihydroxy-3-hydroxymethyl-2-cyclohexen-1-yl]-amino]-α-D-glucopyranosyl}-(1→4)-0-α-D-glucopyranosyl-(1→4)-D-glucopyranose folgender Formel

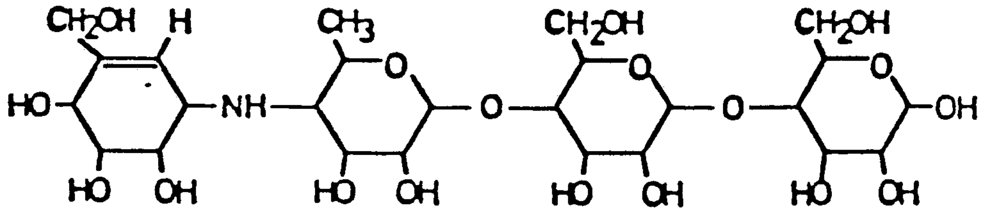

beschrieben in DE-OS-2 347 782.

Die DE-OS-2 719 912 beschreibt ein Reinigungsverfahren für den Saccharaseinhibitor Acarbose, wobei ein wesentlicher Schritt die Chromatographie des vorgereinigten und entsalzten Inhibitors an einem Kationenaustauscher ist. Bei der Überprüfung der handelsüblichen stark sauren Kationenaustauscher hatte es sich gezeigt, daß für die Trennung Kunstharzaustauscher auf Basis Polystyrol mit einem Vernetzungsgrad von 2 - 6 %, vorzugsweise 3 - 4 %, hierzu besonders geeignet sind. Höher vernetzte Austauscher binden Acarbose nur noch geringfügig.

Die GB-A-989 212 beschreibt glasklare, extrem abriebbeständige Perlpolymerisate mit Austauscherfunktionen, die sich in ihrer Morphologie von den erfindungsgemäßen Polymerisaten unterscheiden und damit für die Lösung der erfindungsgemäßen Aufgabe ungeeignet sind.

Ullmann's Enzyklopädie der technischen Chemie, 4. Auflage, 1980, Seiten 2 bis 4 beschreibt allgemein Kondensationsprodukte von Acryl- und Methacrylamid mit Formaldehyd.

Die in DE-A-2 719 912 genannten Austauscher sind gelförmige Austauscher, die gegenüber festen makroporösen Austauschern schlechtere Trennleistungen aufweisen.

Es wurde nun überraschend gefunden, daß das erfindungsgemäße Polymerisat, ein spezieller, makroporöser Kationenaustauscher, trotz hohem Vernetzungsgrad mit besonderem Vorteil für die technischen Trennverfahren von Acarbose Verwendung finden kann.

Die Erfindung betrifft daher makroporöse vernetzte Perlpolymerisate auf der Basis von Aromaten mit einer und mit mehreren Vinylgruppe(n) und hydrophilen Monomeren, erhältlich durch radikalische Polymersation von Aromaten mit einer oder mehreren Vinylgruppen und hydrophilen Monomeren in Gegenwart eines Lösungsmittels für die Monomeren, das aber ein Fällungsmittel bzw. Inertmittel für das entstehende vernetzte Polymerisat ist, und anschließende Sulfierung des isolierten Perlpolymerisats in Gegenwart eines Quellungsmittels für das Polymerisat.

Bevorzugt sind Polymerisate auf der Basis Styrol, Divinylbenzol und Methoxymethylmethacrylsäureamid, Oxethyl(meth)acrylsäureamid(ester), Dimethylaminoethyl(meth)acrylsäureamid(ester) oder Oxypropyl(meth)acrylsäureamid(ester).

Besonders bevorzugt sind Polymerisate, die aus einem Polymerisationsansatz erhältlich sind, in dem 5 bis 25 Gew.-% Methoxymethylmethacrylsäureamid und 6 bis 20 Gew.-% Divinylbenzol, beides bezogen auf die Summe der Monomeren, enthalten sind.

Weiterhin bevorzugt sind solche Polymerisate, in denen mindestens 90 % der im Polymerisat enthaltenen aromatischen Kerne eine Sulfogruppe enthalten.

Als besondere Vorteile des neuen Austauschers seien angeführt:

1. Eine sehr hohe Trennschärfe für die einzelnen Homologen und Verunreinigungen des Inhibitors bei einem vergleichsweise recht groben Korn von 0,1 - 0,4 mm.
2. Eine sehr geringe Volumenänderung des Austauscherbettes während der Arbeits- und Regenerationsphasen des Austauschers.
3. Die harten, widerstandfähigen Körner des Austauschers bewirken zusammen mit ihrer geringen Volumenänderung eine sehr große Stabilität des einmal aufgeschütteten Austauscherbettes in der Säule und damit ein gleichbleibendes Trennverhalten.
   Durchfluß und Druckabfall bleiben während vieler Arbeits- und Regenerationsphasen konstant.
4. Die Elution des Inhibitors aus dem neuen Austauscher erfolgt rascher und mit kleineren Mengen der Elutionslösung als bei den handelsüblichen gelförmigen Kationenaustauschern mit geringer Vernetzung. Für den technischen Einsatz bedeutet dies sowohl eine Einsparung an Zeit, entionisiertem Wasser und für die Elution benötigter Chemikalien als auch eine Energie- und Arbeitseinsparung bei der nachfolgend notwendigen Konzentrierung der wirkstoffhaltigen Fraktion. Der in den Beispielen 1a) bis 1c) durchgeführte Vergleich verschiedener Ionenaustauscher zeigt, daß sowohl die Laufzeit der Säule als auch das Volumen der Substanz enthaltenden Fraktionen auf etwa 1/3 verringert werden kann.

Die Erfindung wird weiterhin beschrieben unter Hinweis auf die Zeichnungen, in denen

Fig. 1 eine graphische Darstellung der gel-chromatographischen Charakteristik des erfindungsgemäßen Polymerisates zeigt;
Fig. 2 ein chromatographisches Diagramm von unreiner Acarbose auf verschiedenen Ionenaustauscherharzen (Beispiel 2) darstellt;
Fig. 3 das Diagramm der Chromatographie von unreiner Acarbose an Ionenaustauscherharzen gemäß der vorliegenden Erfindung (Beispiel 3) zeigt;
Fig. 4 die Reinigung einer Fermentationslösung zeigt; und
Fig. 5 ein Beispiel für die Reinigung im technischem Maßstab darstellt.

Daß es sich bei dem erfindungsgemäßen Polymerisat bzw. Kationenaustauscher um eine Besonderheit handelt, wird insbesondere aus dem gelchromatographischen Verhalten deutlich. Bedingt durch die Morphologie weist das Polymerisat ein ganz besonders breites Plateau für Molekulargewichte im Bereich zwischen 40 000 und 50 000 auf.

Aus der Fig. 1 bei der lg M (Molekulargewicht) gegen R aufgetragen wurde, ist das gut zu ersehen.

$R = 100 \times K$
$K = \frac{VE - VZ}{V_P}$
$V_E$ = Elutionsvolumen
$V_Z$ = Zwischenkornvolumen
$V$ = Porenvolumen.

In Fig. 2 werden Austauscherharze nach dem Stand der Technik (b, c) mit einem Austauscher auf der Basis des erfindungsgemäßen Polymerisats (a) an unreiner Acarbose chromatographisch verglichen.

Die vorteilhafte Struktur des Polymerisates kommt auch im Auswaschverhalten nach der Regeneration mit Salzsäure zum Ausdruck (Beispiel 2a, 2b, 2c). Die Wassermenge liegt im Schnitt um 50 % unter der, die bei konventionellen, käuflich erwerbbaren Harzen üblich ist. Dies ist im Vergleich zu den niedriger vernetzten, gelförmigen Harzen überraschend.

Weiterer Gegenstand der Erfindung ist ein Verfahren zur Herstellung der erfindungsgemäßen Polymerisate, das darin besteht, daß man Aromaten mit einer oder mehreren Vinylgruppen und hydrophile Monomere radikalisch in Suspension unter Zusatz eines Lösungsmittels für die Monomeren, das als Fällungsmittel für das entstehende Polymere wirkt, polymerisiert, das Polymere isoliert und unter Quellung sulfiert.

Als Lösungsmittel für die Monomeren und Fällungsmittel für das vernetzte Polymere werden z. B. aliphatische Kohlenwasserstoffe oder aliphatische oder cycloaliphatische Alkohole eingesetzt.

Für die Sulfierung des makroporösen Perlpolymerisats werden Quellungsmittel eingesetzt.

Die erfindungsgemäßen Polymerisate eignen sich hervorragend zur Reinigung des Saccharaseinhibitors Acarbose.

Die Herstellung der erfindungsgemäßen Polymerisate kann beispielsweise wie im folgenden beschrieben durchgeführt werden.

## **Beispiel 1** (Polymerisatherstellung)

### a) Polymerisation

In einem Sulfierbecher von 3 Liter Inhalt, mit Planschliffdeckel, versehen mit mehreren Öffnungen für Rührer, Thermometer und Stromstörer wird eine wäßrige Flotte vorgelegt. Sie besteht aus 1500 ml Edelwasser in dem 0,07 g Natriumnitrit, 15 g $Na_2HPO_4 \cdot 12H_2O$ und 22,5 g Polyvinylalkohol, mit einem Verseifungsgrad von etwa 80 %, gelöst wurden. Zu der Flotte wird das Monomerengemisch folgender Zusammensetzung gegeben: 261,5 g Styrol, 38,5 g Divinylbenzol technisch (DVB-Gehalt 62,36 %), 15 g Methoxymethylmethacrylsäureamid und 240 g Isododecan. In der Monomermischung wurden zuvor 2,5 g Benzoylperoxid, mit 25 Gew.-% Wasser phlegmatisiert, gelöst. Durch starkes Rühren mit einem Gitterrührer wurde bei 300 Umdrehungen pro Minute die organische Phase in der wäßrigen Flotte fein verteilt. Dann wird die Temperatur auf 65°C erhöht und 5 Stunden gehalten. Anschließend wird die Temperatur auf 75°C erhöht und 1,5 Stunden gehalten. Danach wird 6 Stunden bei 90°C auspolymerisiert. Die fertigen Perlen werden auf einer Glasfilternutsche von der Flotte abgetrennt und mit Edelwasser gewaschen bis der Suspensionsstabilisator ausgespült ist. Bei 75°C wird im Vakuumtrockenschrank über 24 Stunden getrocknet. Ausbeute 346 g Perlpolymerisat.

### b) Sulfierung

In einem 5 Liter Sulfierbecher versehen mit Rührer Thermometer und Rückflußkühler werden 2941 ml konz.

$H_2SO_4$ vorgelegt und mit 131 ml Edelwasser verdünnt. Dann werden 346 g des oben beschriebenen perlpolymerisates zugegeben. Unter ständigem Rühren werden 173 ml Ethylenchlorid zugetropft. Nach vollständiger Zugabe wird 1 Stunde nachgerührt, um eine ausreichende Quellung der Perlen zu erzielen. Anschließend wird die Temperatur innerhalb von 1 Stunde auf 80°C erhöht dort 1/2 Stunde gehalten, um dann weiter auf 100°C erhöht zu werden. Nach 2 Stunden wird über einen absteigenden Kühler das Ethylenchlorid abdestilliert. Letzte Reste des Quellungsmittels werden durch Einblasen von Stickstoff ausgetrieben. Zur vollständigen Sulfierung wird abschließend für 2 Stunden auf 120°C aufgeheizt.

Über Nacht wird abkühlen gelassen. Die Schwefelsäure wird mit einem Siebsteigrohr abgezogen. Die sulfierten Perlen werden einmal mit 75 %-iger $H_2SO_4$ aufgerührt. Die Säure wird wieder abgezogen und durch 45 %-ige $H_2SO_4$ ersetzt. Nach Abziehen dieser Säure wird mit Edelwasser aufgenommen. Ausbeute 2360 ml des Harzes.

Das Ausspülen der restlichen Schwefelsäure erfolgt kontinuierlich in einem Filterrohr, in das die Perlen überführt werden. Durch Flotation wurde von Feinstanteilen abgetrennt. Durch Sedimentation im Filterrohr, nach einer 150 %-igen Bettstreckung, wurden die zu großen Perlen in den unteren Teil des Filterrohres abgesetzt. Die gewünschte Korngröße wurde durch Abschnüffeln mit einem Siliconschlauchheber von oben her abgetragen. Das so erhaltene Harz wurde zur Füllung der Chromatographiesäule verwendet.

Die Beispiele 2 bis 5 sind Reinigungsbeispiele.

**Beispiel 2a**

Eine Säule mit 2,6 cm Durchmesser und 40 cm Höhe (Pharmacia K 26/40) wird mit einer Suspension des erfindungsgemäßen Kationenaustauschers (nach vorhergehendem Herstellungsbeispiel), Korngröße 0,2 - 0,4 mm, in üblicher Weise gefüllt, so daß weder Lufteinschlüsse noch größere Strömungen in der Säule auftreten. Obwohl der Austauscher bereits in der H+-Form vorlag, wurde die Säule noch mit 200 ml 1N-HCL gespült und mit Wasser nachgewaschen, so daß im Ablauf pH-Werte über 4 erreicht wurden.

Die Säule wurde mit 50 ml einer wäßrigen Lösung, welche 1,0 g Acarbose (Komponente 3) 0,5 g Komponente 4, dem um 1 Glucoseeinheit größeren Homologen der Acarbose und 0,2 g Komponente 2, dem um 1 Glucoseeinheit kleineren Homologen der Acarbose beschickt. Die Komponenten enthielten auch noch kleinere Mengen an Verunreinigungen.

Der Durchfluß betrug 66,4 ml/h (12,5 cm/h).

Nach dem Auftragen der Substanz wurde die Säule mit etwa 100 ml wasser gewaschen. Die Elution erfolgte mit 0,025 N-Salzsäure. Das Effluat wurde über ein Differentialrefraktometer (Knauer) als Detektor geleitet und fraktioniert aufgefangen. Die einzelnen Maxima wurden durch Dünnschichtchromatographie identifiziert und die Volumina gemessen.

Der Verlauf der Trennung ist in der Fig. 2, Kurve a wiedergegeben und die Ergebnisse in der Tabelle 1 zusammengefaßt.

Bei dieser Säule wurde auch die Schrumpfung des Austauschers bei der Regeneration mit 1N-Salzsäure und die Quellung bei der Waschung mit Wasser beobachtet. Verwendet man als Säulenfüllung das erfindungsgemäße Polymerisat, so ändert sich das Volumen des Harzes nur um 0,6 %.

In der Tabelle 2 ist diese Volumenänderung im Vergleich mit derjenigen der anderen Ionenaustauschertypen angegeben.

**Vergleichsbeispiel 2b**

Der Versuch wurde völlig analog dem Beispiel 2a) durchgeführt, jedoch wurde die Säule mit dem Ionenaustauscher Lewatit® TSW 40, Korngröße 0,1 - 0,3 mm gefüllt. Die Ergebnisse sind in der Fig. 2, Kurve b und in der Tabelle 1 zusammengefaßt.

**Vergleichsbeispiel 2c**

Der Versuch wurde völlig analog dem Beispiel 2a) durchgeführt, jedoch wurde die Säule mit dem Ionenaustauscher Dowex® 50 W-X 4, Korngröße 0,15 - 0,45 mm gefüllt. Die Ergebnisse sind in der Fig. 2, Kurve c und der Tabelle 1 zusammengefaßt.

**Beispiel 3**

Der Versuch wurde analog Beispiel 2a) durchgeführt, jedoch wurde die Säule mit einem feinkörnigen Ionenaustauscher vom erfindungsgemäßen Typ mit den Korngrößen 0,03 - 0,12 mm gefüllt. Bei gleicher Durchflußgeschwindigkeit von 66,3 ml/h ist die Trennung gegenüber der in Beispiel 2a) wesentlich verbessert (Fig. 3). Die Komponenten werden wie im Beispiel 2a) nach fast den gleichen Zeiträumen eluiert, die Maxima sind jedoch steiler und damit verringert sich das jeweilige Volumen der Komponente noch stärker (Tabelle 3).

**Legende zu den Figuren 2 und 3:**

1. Auftragung auf die Säule
2. Nachwaschen der Säule mit Wasser
3. Beginn des Auftragens der 0,025 N-Salzsäure

Der Zeitpunkt 0 wurde auf den Beginn des Ausflusses der Salzsäure aus der Säule gelegt.

**Beispiel 4**

Bei diesem Beispiel wird eine Acarbose-haltige Lösung eingesetzt, welche aus dem Fermentationsansatz nach den Stufen 1 - 5 der Beschreibung der DE-OS-2 719 912 gewonnen wurde. Insgesamt wurden 1300 ml der Lösung mit einem Gehalt von 5,3 g Acarbose auf eine Säule von 26 mm Ø und einer Betthöhe von 377 mm aufgetragen. In der Säule befanden sich 200 ml des erfindungsgemäßen Kationenaustauschers in einer Korngröße von 0,1 - 0,25 mm. Die Geschwindigkeit der Auftragung betrug 200 ml/h. Nach Beendigung der Auftragung wurde die Säule mit entionisiertem Wasser nachgespült und anschließend mit 0,025 N-Salzsäure mit einer Geschwindigkeit von 100 ml/h eluiert. Die mit dem Differentialrefraktometer erhaltene Elutionskurve ist in Fig. 4 dargestellt. Es wurden 630 ml Hauptfraktion erhalten, die nach Neutralisation mit Lewatit® MP 62 (Basenform) und Gefriertrocknung 4,19 g Acarbose ergab (Tabelle 4). Die Reinheit der Acarbose betrug 89,5 %.

**Beispiel 5**

Eine Rohlösung von 38 $m^3$, gewonnen aus einem Fermentationsansatz und den nachfolgenden Reinigungsstufen 1 - 5 der Beschreibung der DE-OS-2 719 912 mit einem Gehalt von 144 kg Acarbose, wurde mit einer Geschwindigkeit von 4000 Ltr./h auf folgende Säule aufgetragen: Durchmesser 250 cm, zylindrische Höhe 300 cm gefüllt mit 11 $m^3$ erfindungsgemäßes Polymerisat in der $H^+$-Form. Nach dem Auftragen wurde die Säule mit entionisiertem Wasser nachgespült. Die Säule wurde mit 2000 Ltr/h mit einem in Stufen ansteigenden Salzsäuregradienten eluiert, und zwar 8 Stunden mit 0,01 N-HCl, 12 Stunden mit 0,02 N-HCl und 10 Stunden mit 0,03 N-HCl. Der Fortgang der Elution wurde mit einem Differentialrefraktometer gemessen (Fig. 5) und die Hauptfraktion entsprechend der Kurve geschnitten. Die Hauptfraktion von 23 500 Ltr. enthielt 114 kg Acarbose, das sind 79 % des Einsatzes.
(In Fig. 5 und den übrigen Zeichnungen bedeutet "Komp." Komponente).

**Tabelle 1** Vergleich der Ionenaustauscher zur Reinigung von Acarbose

| Elution von | Beispiel 2c Dowex® 50 WX 4 | | | Beispiel 2b Lewatit® TSW 40 | | | Beispiel 2a erfindungsgemäßes Polymerisat | | |
|---|---|---|---|---|---|---|---|---|---|
| | Elutions-Volumen ml | Zeit h | Fraktions-Volumen ml | Elutions-Volumen ml | Zeit h | Fraktions-Volumen ml | Elutions-Volumen ml | Zeit h | Fraktions-Volumen ml |
| Komponent 4 | 2512 | 44 | 728 | 2160 | 35 | 779 | 570 | 9,3 | 248 |
| Komponent 3 | 3320 | 56,5 | 988 | 2985 | 48,5 | 891 | 895 | 15 | 327 |
| Komponent 2 | 4824 | 83 | - | 3877 | 63 | 869 | 1492 | 25 | 405 |

Das Elutionsvolumen und die Elutionszeit wurden bestimmt vom Beginn der Elution mit 0,025 N-Salzsäure bis zum Maximum der Refraktometerkurve der jeweiligen Komponente.

**Tabelle 2** Volumenänderung der Ionenaustauscher beim Übergang von 1 N-Salzsäure auf Wasser

| Ionenaustauscher | Korngröße mm | Volumenänderung % |
| --- | --- | --- |
| Dowex® 50 WX 4 | 0,15 - 0,45 | 18,4 |
| Lewatit® TSW 40 | 0,1 - 0,3 | 13,0 |
| erfindungsgemäß | 0,2 - 0,4 | 0,6 |

Die Volumenänderung wurde am gepackten Bett in einer Säule mit 26 mm Durchmesser und 40 cm Füllhöhe gemessen.

**Tabelle 3** Elution der Komponenten aus Beispiel 3

| Elution | Elutionsvolumen ml | Elutionszeit h | Fraktionsvolumen ml |
| --- | --- | --- | --- |
| Komponente 4 | 605 | 9,5 | 134 |
| Komponente 3 | 935 | 14,5 | 234 |
| Komponente 2 | 1630 | 25,5 | 268 |

**Tabelle 4** Ergebnis einer Acarbose-Reinigung im Laboratorium

| | | Volumen | Aktivität | | Ausbeute | |
| --- | --- | --- | --- | --- | --- | --- |
| | | ml | SIE/ml | SIE | % | g*) |
| Aufgetragenes Desorbat **) | | 1300 | 317 | 412100 | = 100 | |
| Durchfluß der Trennsäule | | 1320 | 0,4 | 500 | 0,1 | |
| Nachspülung der Trennsäule | | 880 | 0,1 | 90 | 0,0 | |
| Elution | Vorfraktionen 15-46 | 465 | 31,1 | 14460 | 3,5 | 2,09 |
| | Hauptfraktion 47-89 | 630 | 538 | 338940 | 82,2 | 4,19 |
| | Nachfraktion 90-117 | 405 | 6,6 | 2673 | 0,7 | 0,74 |
| | | | | | 86,5 | |

*) Nach Neutralisation mit Lewatit® MP 62 und Gefriertrocknung.
**) hergestellt nach der DE-OS 2 719 912.

**Patentansprüche**

1. Makroporöses vernetztes Perlpolymersat, erhältlich durch Polymerisation von Aromaten mit einer und mit mehreren Vinylgruppe(n) und hydrophilen Monomeren in Gegenwart eines Lösungsmittels für die Monomeren, das als Fällungsmittel für das entstehende vernetzte Polymerisat wirkt, Isolierung des Perlpolymerisats und dessen anschließende Sulfierung in Gegenwart eines Quellungsmittels für das Polymerisat.

2. Polymerisat nach Anspruch 1, dadurch gekennzeichnet, daß der Polymerisationsansatz 5 bis 25 Gew.-% Methoxymethylmethacrylsäureamid und 6 bis 20 Gew.-% Divinylbenzol, beides bezogen auf die Summe der Monomeren, enthält.

3. Polymerisat nach den Ansprüchen 1 und 2, dadurch gekennzeichnet, daß mindestens 90 % der im Polymerisat enthaltenen aromatischen Kerne eine Sulfogruppe enthalten.

4. Polymerisat nach den Ansprüchen 1, 2 und 3 erhältlich durch Verwendung von aliphatischen oder cycloaliphatischen Alkoholen oder aliphatischen Kohlenwasserstoffen, die als Lösungsmittel für die Monomeren, aber als Fällungsmittel für das vernetzte Polymere wirken.

5. Polymerisat nach den Ansprüchen 1, 2, 3 und 4, erhältlich durch Sulfierung des makroporösen Polymerisates in Gegenwart eines Quellungsmittels.

6. Polymerisat nach den Ansprüchen 1 bis 5 in Perlform.

7. Verfahren zur Herstellung von makroporösen vernetzten Perlpolymerisaten, die sich zur Reinigung von Acarbose eignen, dadurch gekennzeichnet, daß man Aromaten mit einer Vinylgruppe, Aromaten mit mehreren Vinylgruppen und hydrophile Monomere radikalisch in Suspension unter Zusatz eines Lösungsmittels für die Monomeren, das als Fällungsmittel für das entstehende Polymere wirkt, polymerisiert, das Polymere isoliert und unter Quellung sulfiert.

8. Verfahren zur Herstellung von Polymerisaten nach Anspruch 7, dadurch gekennzeichnet, daß man Styrol, Divinylbenzol und Methoxymethylmethacrylsäureamid in Gegenwart eines Lösungsmittels für die Monomeren, das ein Fällungsmittel für das entstehende Polymerisat ist, polymerisiert, isoliert und anschließend in einem

Quellungsmittel sulfiert.

9. Verwendung von Polymerisaten nach den Ansprüchen 1 bis 6 zur Reinigung von Acarbose.

10. Verwendung von makroporösen vernetzten Perlpolymerisaten, erhältlich durch Polymerisation von Styrol, Divinylbenzol und Methoxymethylmethacrylsäureamid in Gegenwart eines Lösungsmittels für die Monomeren, das ein Fällungsmittel für das entstehende Polymerisat ist, Isolierung des Polymerisats und dessen anschließende Sulfierung in Gegenwart eines Quellungsmittels für das Polymerisat, zur Reinigung von Acarbose.

## Claims

1. Macroporous crosslinked bead polymer obtainable by polymerisation of aromatic compounds possessing one or more vinyl group(s) and hydrophilic monomers in the presence of a solvent for the monomers which acts as precipitant for the crosslinked polymer formed, isolation of the bead polymer and its subsequent sulphonation in the presence of a swelling agent for the polymer.

2. Polymer according to Claim 1, characterised in that the polymerisation mixture contains 5 to 25 % by weight of methoxymethylmethacrylamide and 6 to 20 % by weight of divinylbenzene, both relative to the total of the monomers.

3. Polymer according to Claims 1 and 2, characterised in that at least 90 % of the aromatic nuclei present in the polymer contain a sulpho group.

4. Polymer according to Claims 1, 2 and 3, obtainable by the use of aliphatic or cycloaliphatic alcohols or aliphatic hydrocarbons which act as solvents for the monomers but as precipitants for the crosslinked polymer.

5. Polymer according to Claims 1, 2, 3 and 4, obtainable by sulphonation of the macroporous polymer in the presence of a swelling agent.

6. Polymer according to Claims 1 to 5 in bead form.

7. Process for the preparation of macroporous crosslinked bead polymers which are suitable for the purification of acarbose, characterised in that aromatic compounds possessing a vinyl group, aromatic compounds possessing several vinyl groups and hydrophilic monomers are subjected to free-radical polymerisation in a suspension to which a solvent for the monomers is added, which solvent acts as precipitant for the polymer formed, and the polymer is isolated and sulphonated with swelling.

8. Process for the preparation of polymers according to Claim 7, characterised in that styrene, divinylbenzene and methoxymethylmethacrylamide are polymerised in the presence of a solvent for the monomers which is a precipitant for the polymer formed, the polymer is isolated and subsequently sulphonated in a swelling agent.

9. Use of polymers according to Claims 1 to 6 for the purification of acarbose.

10. Use, for the purification of acarbose, of macroporous crosslinked bead polymers obtainable by polymerisation of styrene, divinylbenzene and methoxymethylmethacrylamide in the presence of a solvent for the monomers which is a precipitant for the polymer formed, isolation of the polymer and its subsequent sulphonation in the presence of a swelling agent for the polymer.

## Revendications

1. Polymère en perles macroporeux réticulé, obtenu par polymérisation de composés aromatiques à un et à plusieurs groupes vinyle et de monomères hydrophiles en présence d'un solvant des monomères agissant comme agent précipitant pour le polymère réticulé formé, isolement du polymère en perles et sulfonation subséquente de ce polymère en présence d'un agent gonflant du polymère.

2. Polymère selon la revendication 1, caractérisé en ce que le mélange de polymérisation contient 5 à 25 % en poids de méthoxyméthylméthacrylamide et 6 à 20 % en poids de divinylbenzène, dans les deux cas par rapport à la somme des monomères.

3. Polymère selon les revendications 1 et 2, caractérisé en ce que 90 % au moins des noyaux aromatiques contenus dans le polymère portent un groupe sulfo.

4. Polymère selon les revendications 1, 2 et 3, obtenu par utilisation d'alcools aliphatiques ou cycloaliphatiques ou hydrocarbures aliphatiques servant de solvants pour les monomères mais d'agents précipitants pour le polymère réticulé.

5. Polymère selon les revendications 1, 2, 3 et 4, obtenu par sulfonation du polymère macroporeux en présence d'un agent gonflant.

6. Polymère selon les revendications 1 à 5, sous forme de perles.

7. Procédé de préparation de polymères en perles macroporeux réticulés convenant pour la purification de l'acarbose, caracterisé en ce que l'on polymérise des composes aromatiques à un groupe vinyle, des composés aromatiques à plusieurs groupes vinyle et des monomères hydrophiles par polymérisation radicalaire en suspension avec adjonction d'un solvant pour les monomères servant d'agent précipitant pour le polymère formé, on isole le polymère et on le sulfone à l'etat gonflé.

8. Procédé de préparation de polymères selon la revendication 7, caractérisé en ce que l'on polymérise du

styrène, du divinylbenzène et du méthoxyméthylméthacrylamide en présence d'un solvant des monomères qui est un agent précipitant du polymère formé, on isole le polymère puis on le sulfone dans un agent gonflant.

9. Utilisation des polymères selon les revendications 1 à 6 pour la purification de l'acarbose.

10. Utilisation de polymères en perles réticulés macroporeux obtenus par polymérisation du styrène, du divinylbenzène et du méthoxyméthylméthacrylamide en présence d'un solvant des monomères qui est un agent précipitant pour le polymère formé, isolement du polymère et sulfonation de celui-ci en présence d'un agent gonflant de ce polymère, pour la purification de l'acarbose.

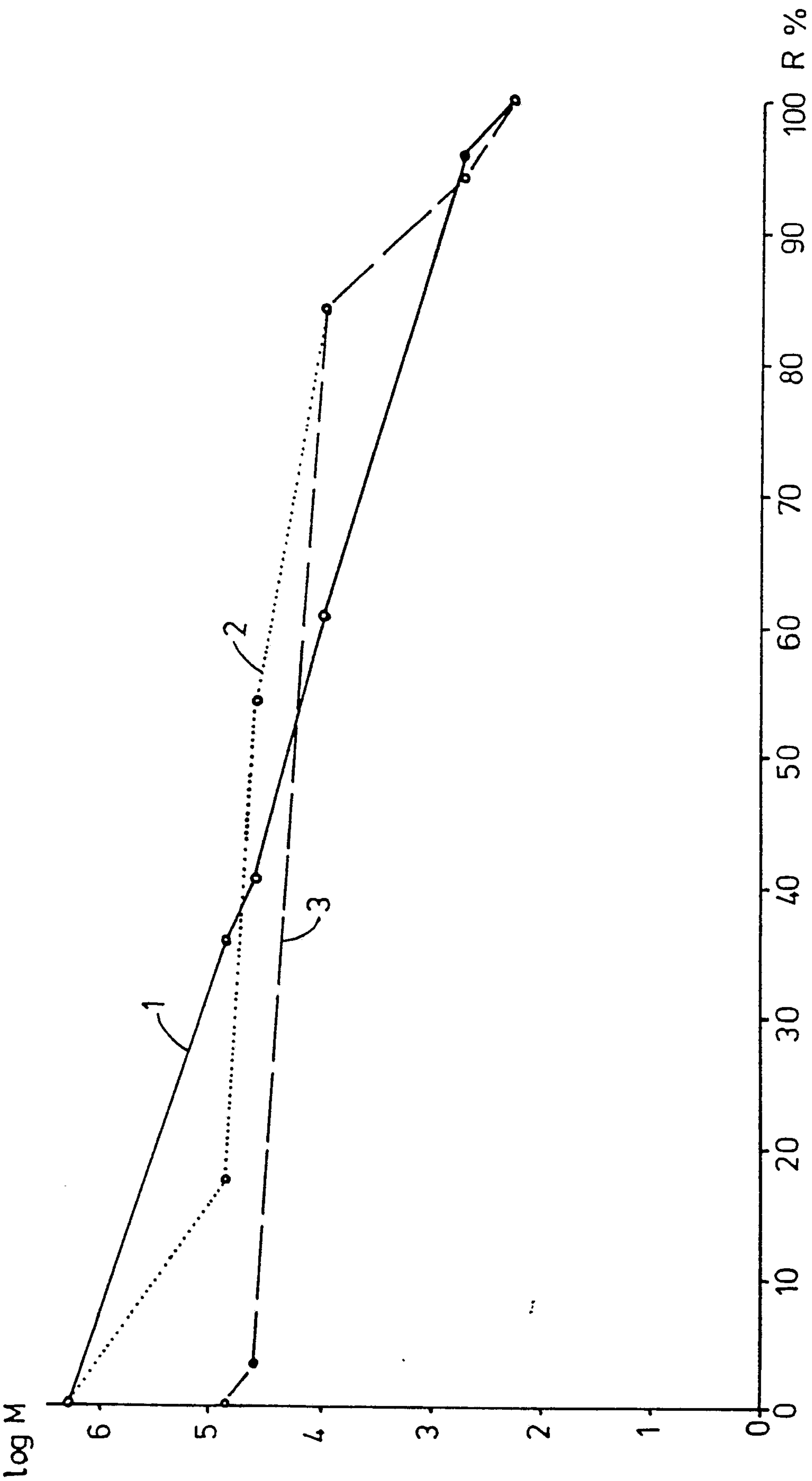


FIG. 1

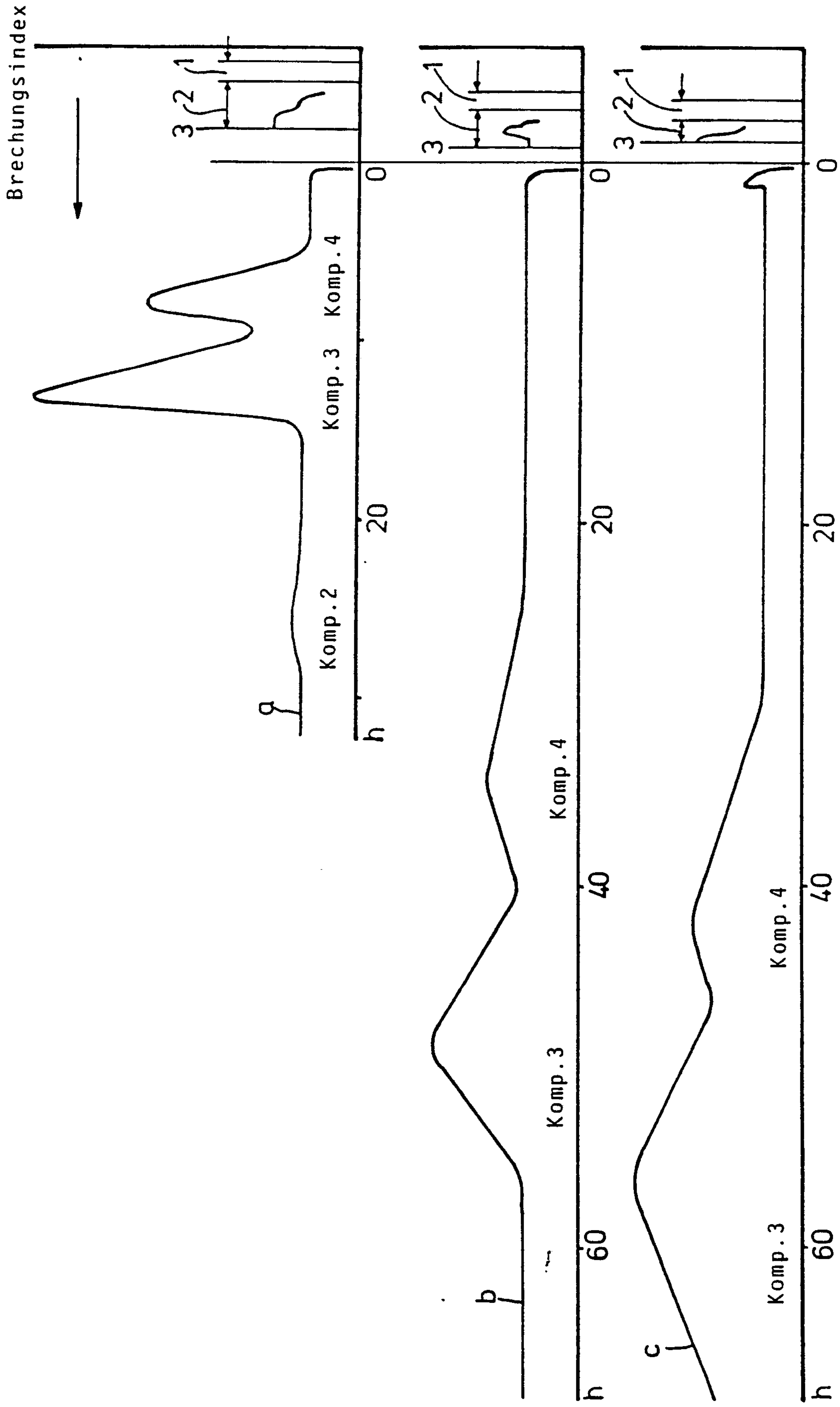


FIG. 2

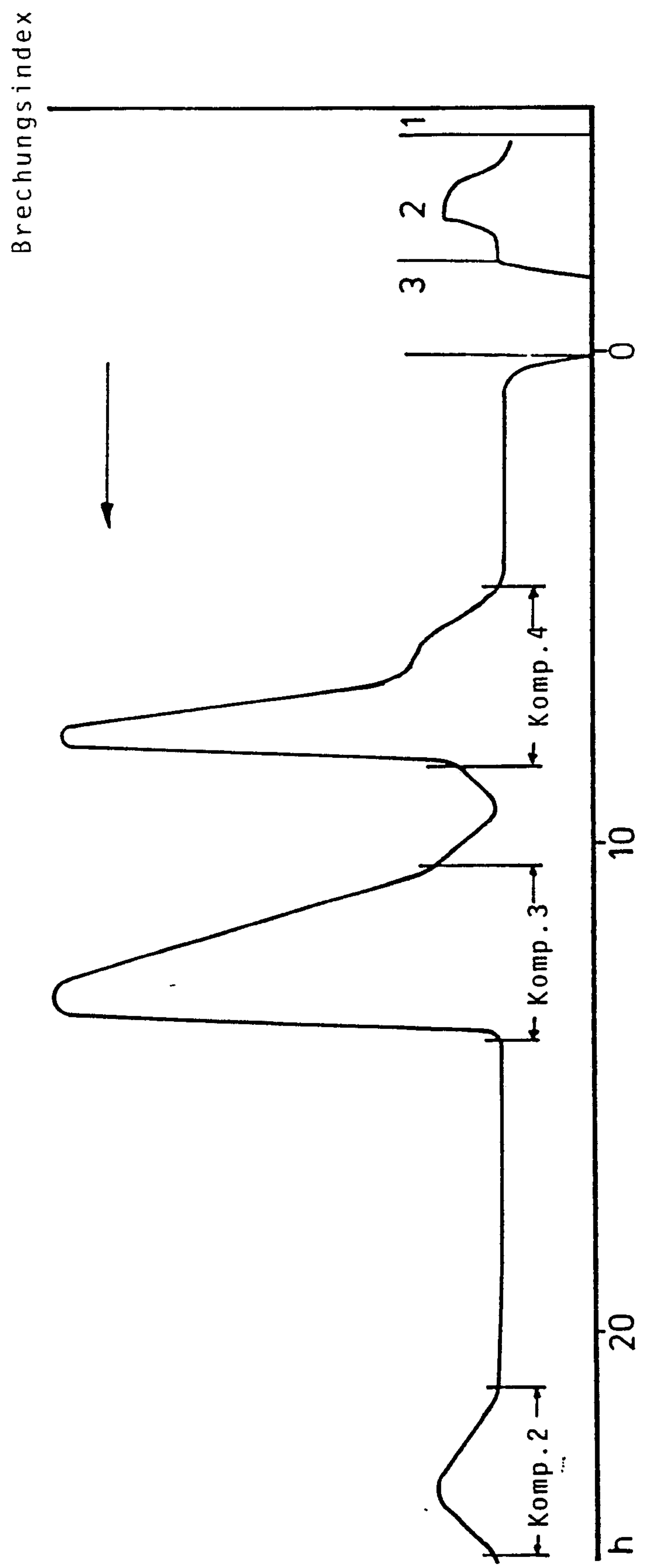


FIG. 3

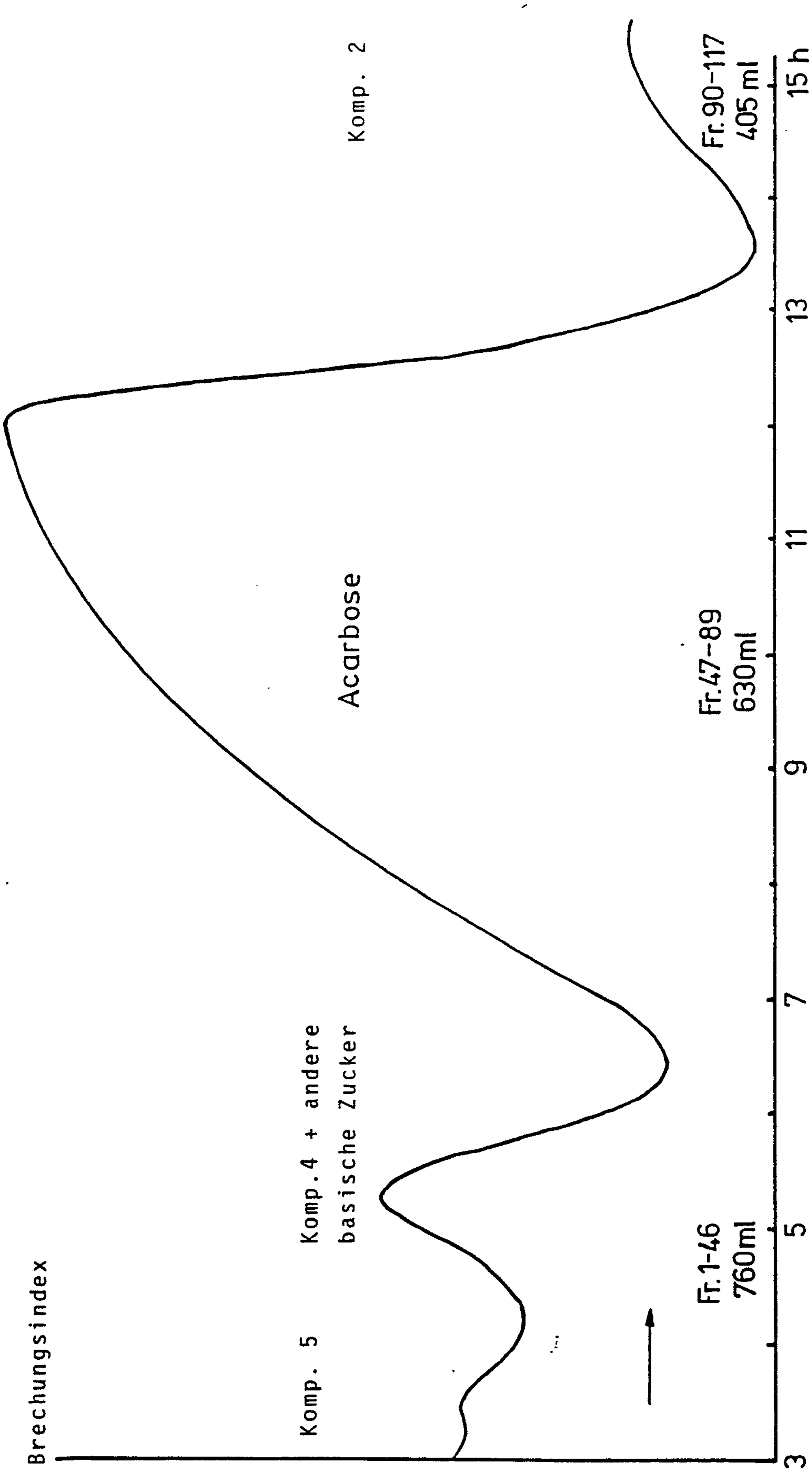

Brechungsindex
Komp. 5
Komp. 4 + andere basische Zucker
Acarbose
Komp. 2
Fr. 1-46 760ml
Fr. 47-89 630ml
Fr. 90-117 405ml
3
5
7
9
11
13
15 h


FIG. 4

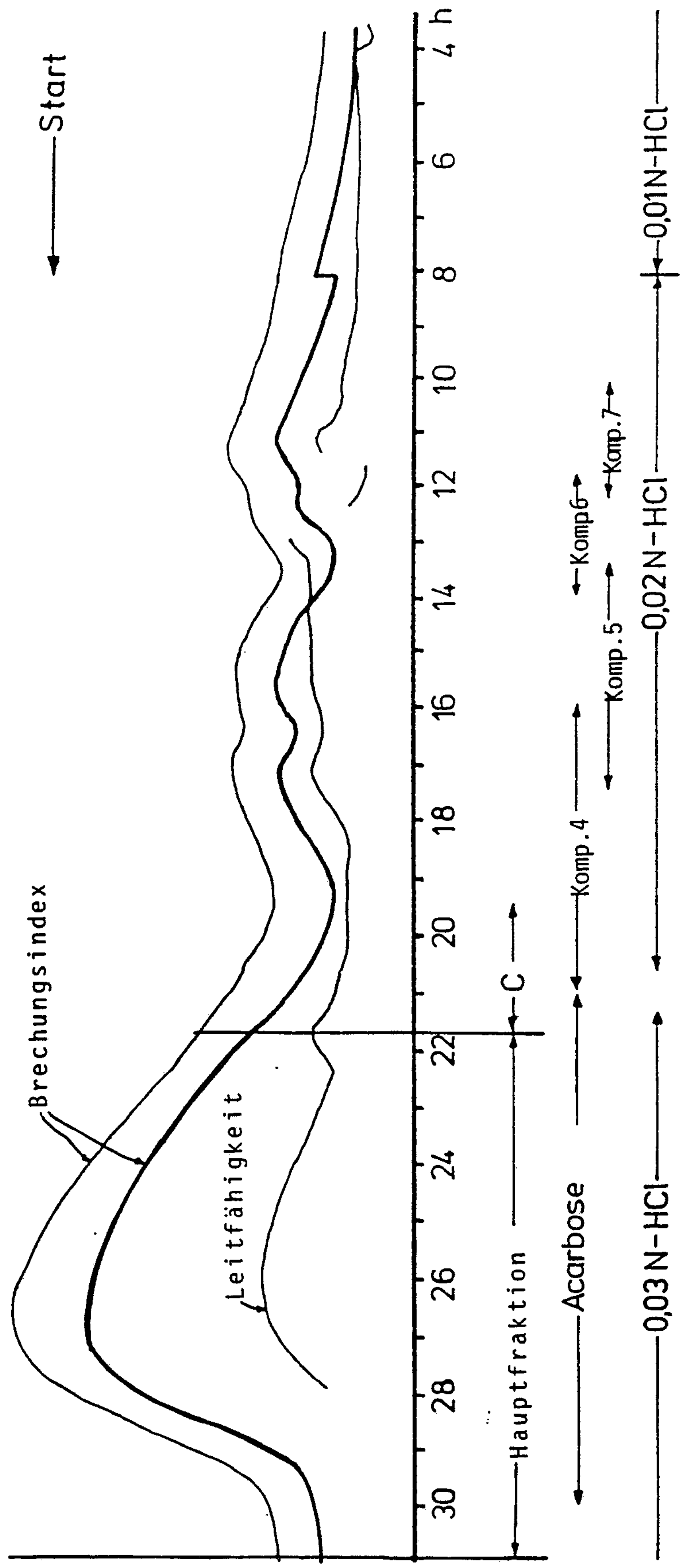

Start
Brechungsindex
Leitfähigkeit
4 h
6
8
10
12
14
16
18
20
22
24
26
28
30
C
Hauptfraktion
Komp6
Komp.7
Komp.5
Komp.4
Acarbose
0,01N-HCl
0,02N-HCl
0,03N-HCl


FIG. 5